# EUROPEAN PATENT APPLICATION

(11) **EP 1 367 125 A1**
(43) Date of publication of application: **03.12.2003**
(21) Application number: 02291316.4
(22) Date of filing: 30.05.2002
(51) Int. Cl.: C12N 15/62

(54) **Vectors for expression of biotinylated proteins in mammalian cells, and their use for identification of protein-nucleic acid interactions in vivo**

(71) Applicant: CENTRE NATIONAL DE LA RECHERCHE SCIENTIFIQUE (CNRS), 75016 Paris (FR)
(72) Inventor: Ogryzko, Vasily, 92260 Fontenay-aux-Roses (FR); Lehrman, Heike, 91700 Ste Genevieve des Bois (FR); Gilbert, Christele, 94150 Rungis (FR); Viens, Antoine, 94240 l'Hay-les-Roses (FR); Mechold, Undine, 92260 Fontenay-aux-Roses (FR); Harel-Bellan, Annick, 75005 Paris (FR)
(74) Representative: Warcoin, Jacques

(57) **Abstract**

A method for expressing in eucaryote cells a modified target protein of interest comprising expressing (i) a protein of interest and (ii) a modifying enzyme that achieves the modification of said target protein, wherein the target protein of interest and the modifying enzyme are coexpressed from the same RNA.

LISTE DE SEQUENCES

<110> CNRS Centre National de la Recherche Scientifique

<120> Vectors for expression of biotinylated proteins in mammalian cells

<130> D20142

<160> 2

<170> PatentIn Ver. 2.1

<210> 1
<211> 9037
<212> DNA
<213> Artificial sequence

<220>
<223> Description of artificial sequence : nucleotidic sequence of vector pBBHN

<400> 1
   ccacgcttcc cgaagggaga aaggcggaca ggtatccggt aagcggcagg gtcggaacag 60
   gagagcgcac gagggagctt ccagggggaa acgcctggta tctttatagt cctgtcgggt 120
   ttcgccacct ctgacttgag cgtcgatttt tgtgatgctc gtcagggggg cggagcctat 180
   ggaaaaacgc cagcaacgcg gcctttttac ggttcctggc cttttgctgg ccttttgctc 240
   acatgttctt tcctgcgtta tcccctgatt ctgtggataa ccgtattacc gcctttgagt 300
   gagctgatac cgctcgccgc agccgaacga ccgagcgcag cgagtcagtg agcgaggaag 360
   cggaagagcg cccaatacgc aaaccgcctc tccccgcgcg ttggccgatt cattaatgca 420
   gctggcacga caggtttccc gactggaaag cgggcagtga gcgcaacgca attaatgtga 480
   gttagctcac tcattaggca ccccaggctt tacactttat gcttccggct cgtatgttgt 540
   gtggaattgt gagcggataa caatttcaca caggaaacag ctatgaccat gattacgcca 600
   agctttgctc ttaggagttt cctaatacat cccaaactca aatatataaa gcatttgact 660
   tgttctatgc cctagggggc ggggggaagc taagccagct ttttttaaca tttaaaatgt 720
   taattccatt ttaaatgcac agatgttttt atttcataag ggtttcaatg tgcatgaatg 780
   ctgcaatatt cctgttacca aagctagtat aaataaaaat agataaacgt ggaaattact 840
   tagagtttct gtcattaacg tttccttcct cagttgacaa cataaatgcg ctgctgagaa 900
   gccagtttgc atctgtcagg atcaatttcc cattatgcca gtcatattaa ttactagtca 960
   attagttgat ttttattttt gacatataca tgtgaaagac cccacctgta ggtttggcaa 1020
   gctagcttaa gtaacgccat tttgcaaggc atggaaaaat acataactga gaatagaaaa 1080
   gttcagatca aggtcaggaa cagatggaac agctgaatat gggccaaaca ggatatctgt 1140
   ggtaagcagt tcctgccccg gctcagggcc aagaacagat ggaacagctg aatatgggcc 1200
   aaacaggata tctgtggtaa gcagttcctg ccccggctca gggccaagaa cagatggtcc 1260
   ccagatgcgg tccagccctc agcagtttct agagaaccat cagatgtttc cagggtgccc 1320
   caaggacctg aaatgaccct gtgccttatt tgaactaacc aatcagttcg cttctcgctt 1380
   ctgttcgcgc gcttctgctc cccgagctca ataaaagagc ccacaacccc tcactcggcg 1440
   cgccagtcct ccgattgact gagtcgcccg ggtacccgtg tatccaataa accctcttgc 1500
   agttgcatcc gacttgtggt ctcgctgttc cttgggaggg tctcctctga gtgattgact 1560
   acccgtcagc gggggtcttt catttggggg ctcgtccggg atcgggagac ccctgcccag 1620
   ggaccaccga cccaccaccg ggaggtaagc tggccagcaa cttatctgtg tctgtccgat 1680
   tgtctagtgt ctatgactga ttttatgcgc ctgcgtcggt actagttagc taactagctc 1740
   tgtatctggc ggacccgtgg tggaactgac gagttcggaa cacccggccg caaccctggg 1800
   agacgtccca gggacttcgg gggccgtttt tgtggcccga cctgagtcca aaaatcccga 1860
   tcgttttgga ctctttggtg cacccccctt agaggaggga tatgtggttc tggtaggaga 1920
   cgagaaccta aaacagttcc cgcctccgtc tgaatttttg ctttcggttt gggaccgaag 1980
   ccgcgccgcg cgtcttgtct gctgcagcat cgttctgtgt tgtctctgtc tgactgtgtt 2040
   tctgtatttg tctgaaaata tgggcccggg ccagactgtt accactccct taagtttgac 2100
   cttaggtcac tggaaagatg tcgagcggat cgctcacaac cagtcggtag atgtcaagaa 2160
   gagacgttgg gttaccttct gctctgcaga atggccaacc tttaacgtcg gatggccgcg 2220
   agacggcacc tttaaccgag acctcatcac ccaggttaag atcaaggtct tttcacctgg 2280
   cccgcatgga cacccagacc aggtccccta catcgtgacc tgggaagcct tggcttttga 2340
   cccccctccc tgggtcaagc cctttgtaca ccctaagcct ccgcctcctc ttcctccatc 2400
   cgccccgtct ctcccccttg aacctcctcg ttcgaccccg cctcgatcct ccctttatcc 2460
   agccctcact ccttctctag gcgcccccat atggccatat gagatcctat atggggcacc 2520
   cccgcccctt gtaaacttcc ctgaccctga catgacaaga gttactaaca gcccctctct 2580
   ccaagctcac ttacaggctc tctacttagt ccagcacgaa gtctggagac ctctggcggc 2640
   agcctaccaa gaacaactgg accgaccggt ggtacctcac ccttaccgag tcggcgacac 2700
   agtgtgggtc cgccgacacc agactaagaa cctagaacct cgctggaaag gaccttacac 2760
   agtcctgctg accaccccca ccgccctcaa agtagacggc atcgcagctt ggatacacgc 2820
   cgcccacgtg aaggctgccg accccggggg tggaccatcc tctagactgc cagatctgct 2880
   atggccggac ttaatgatat ttttgaagcc cagaagattg aatggcatga actcgaccat 2940
   caccatcacc accatctcga gatgcatgcg gccgcctgca ggtcgagccg gcgggtttct 3000
   gacatccggc gggtgactca caaccccaga aacagacatc catgggaaag gataacaccg 3060
   tgccactgaa attgattgcc ctgttagcga acggtgaatt tcactctggc gagcagttgg 3120
   gtgaaacgct gggaatgagc cgggcggcta ttaataaaca cattcagaca ctgcgtgact 3180
   ggggcgttga tgtctttacc gttccgggta aaggatacag cctgcctgag cctatccagt 3240
   tacttaatgc taaacagata ttgggtcagc tggatggcgg tagtgtagcc gtgctgccag 3300
   tgattgactc cacgaatcag taccttcttg atcgtatcgg agagcttaaa tcgggcgatg 3360
   cttgcattgc agaataccag caggctggcc gtggtcgccg gggtcggaaa tggttttcgc 3420
   cttttggcgc aaacttatat ttgtcgatgt tctggcgtct ggaacaaggc ccggcggcgg 3480
   cgattggttt aagtctggtt atcggtatcg tgatggcgga agtattacgc aagctgggtg 3540
   cagataaagt tcgtgttaaa tggcctaatg acctctatct gcaggatcgc aagctggcag 3600
   gcattctggt ggagctgact ggcaaaactg gcgatgcggc gcaaatagtc attggagccg 3660
   ggatcaacat ggcaatgcgc cgtgttgaag agagtgtcgt taatcagggg tggatcacgc 3720
   tgcaggaagc ggggatcaat ctcgatcgta atacgttggc ggccatgcta atacgtgaat 3780
   tacgtgctgc gttggaactc ttcgaacaag aaggattggc accttatctg tcgcgctggg 3840
   aaaagctgga taattttatt aatcgcccag tgaaacttat cattggtgat aaagaaatat 3900
   ttggcatttc acgcggaata gacaaacagg gggctttatt acttgagcag gatggaataa 3960
   taaaaccctg gatgggcggt gaaatatccc tgcgtagtgc agaaaaatag cggccggccg 4020
   catcgagaga ccgggagaca caacggtttc cctctagcgg gatcaattcc gccccccccc 4080
   ctaacgttac tggccgaagc cgcttggaat aaggccggtg tgcgtttgtc tatatgttat 4140
   tttccaccat attgccgtct tttggcaatg tgagggcccg gaaacctggc cctgtcttct 4200
   tgacgagcat tcctaggggt ctttcccctc tcgccaaagg aatgcaaggt ctgttgaatg 4260
   tcgtgaagga agcagttcct ctggaagctt cttgaagaca aacaacgtct gtagcgaccc 4320
   tttgcaggca gcggaacccc ccacctggcg acaggtgcct ctgcggccaa aagccacgtg 4380
   tataagatac acctgcaaag gcggcacaac cccagtgcca cgttgtgagt tggatagttg 4440
   tggaaagagt caaatggctc tcctcaagcg tattcaacaa ggggctgaag gatgcccaga 4500
   aggtacccca ttgtatggga tctgatctgg ggcctcggtg cacatgcttt acatgtgttt 4560
   agtcgaggtt aaaaaaacgt ctaggccccc cgaaccacgg ggacgtggtt ttcctttgaa 4620
   aaacacgata ataccatgga ttcatacctg ctgatgtggg gactgctcac gttcatcatg 4680
   gtgcctggct gccaggcaga gctctgtgac gatgacccgc cagagatccc acacgccaca 4740
   ttcaaagcca tggcctacaa ggaaggaacc atgttgaact gtgaatgcaa gagaggtttc 4800
   cgcagaataa aaagcgggtc actctatatg ctctgtacag gaaactctag ccactcgtcc 4860
   tgggacaacc aatgtcaatg cacaagctct gccactcgga acacaacgaa acaagtgaca 4920
   cctcaacctg aagaacagaa agaaaggaaa accacagaaa tgcaaagtcc aatgcagcca 4980
   gtggaccaag cgagccttcc aggtcactgc agggaacctc caccatggga aaatgaagcc 5040
   acagagagaa tttatcattt cgtggtgggg cagatggttt attatcagtg cgtccaggga 5100
   tacaggctct acacagaggt tcctgctgag agcgtctgca aaatgaccca cgggaagaca 5160
   aggtggaccc agccccagct catatgcaca ggtgaaatgg agaccagtca gtttccaggt 5220
   gaagagaagc ctcaggcaag ccccgaaggc cgtcctgaga gtgagacttc ctgcctcgtc 5280
   acaacaacag attttcaaat acagacagaa atggctgcaa ccatggagac gtccatattt 5340
   acaacagagt accaggtagc agtggccggc tgtgttttcc tgctgatcag cgtcctcctc 5400
   ctgagtgggc tcacctggca gcggagacag aggaagagta gaagaacaat ctagaggatc 5460
   cggattagtc caatttgtta aagacaggat atcagtggtc caggctctag ttttgactca 5520
   acaatatcac cagctgaagc ctatagagta cgagccatag ataaaataaa agattttatt 5580
   tagtctccag aaaaaggggg gaatgaaaga ccccacctgt aggtttggca agctagctta 5640
   agtaacgcca ttttgcaagg catggaaaaa tacataactg agaatagaga agttcagatc 5700
   aaggtcagga acagatggaa cagctgaata tgggccaaac aggatatctg tggtaagcag 5760
   ttcctgcccc ggctcagggc caagaacaga tggaacagct gaatatgggc caaacaggat 5820
   atctgtggta agcagttcct gccccggctc agggccaaga acagatggtc cccagatgcg 5880
   gtccagccct cagcagtttc tagagaacca tcagatgttt ccagggtgcc ccaaggacct 5940
   gaaatgaccc tgtgccttat ttgaactaac caatcagttc gcttctcgct tctgttcgcg 6000
   cgcttctgct ccccgagctc aataaaagag cccacaaccc ctcactcggg gcgccagtcc 6060
   tccgattgac tgagtcgccc gggtacccgt gtatccaata aaccctcttg cagttgcatc 6120
   cgacttgtgg tctcgctgtt ccttgggagg gtctcctctg agtgattgac tacccgtcag 6180
   cgggggtctt tcacatgcag catgtatcaa aattaatttg gttttttttc ttaagtattt 6240
   acattaaatg gccatagtac ttaaagttac attggcttcc ttgaaataaa catggagtat 6300
   tcagaatgtg tcataaatat ttctaatttt aagatagtat ctccattggc tttctacttt 6360
   ttcttttatt tttttttgtc ctctgtcttc catttgttgt tgttgttgtt tgtttgtttg 6420
   tttgttggtt ggttggttaa ttttttttta aagatcctac actatagttc aagctagact 6480
   attagctact ctgtaaccca gggtgacctt gaagtcatgg gtagcctgct gttttagcct 6540
   tcccacatct aagattacag gtatgagcta tcatttttgg tatattgatt gattgattga 6600
   ttgatgtgtg tgtgtgtgat tgtgtttgtg tgtgtgattg tgtatatgtg tgtatggttg 6660
   tgtgtgattg tgtgtatgta tgtttgtgtg tgattgtgtg tgtgtgattg tgcatgtgtg 6720
   tgtgtgtgat tgtgtttatg tgtataattg tgtgtgtgtg tgtgtgtgtg tgtgtgtgtg 6780
   tgtgtgtgtg tgtgttgtgt atatatattt atggtagtga gaggcaacgc tccggctcag 6840
   gtgtcaggtt ggtttttgag acagagtctt tcacttagct tggaattcac tggccgtcgt 6900
   tttacaacgt cgtgactggg aaaaccctgg cgttacccaa cttaatcgcc ttgcagcaca 6960
   tccccctttc gccagctggc gtaatagcga agaggcccgc accgatcgcc cttcccaaca 7020
   gttgcgcagc ctgaatggcg aatggcgcct gatgcggtat tttctcctta cgcatctgtg 7080
   cggtatttca caccgcatat ggtgcactct cagtacaatc tgctctgatg ccgcatagtt 7140
   aagccagccc cgacacccgc caacacccgc tgacgcgccc tgacgggctt gtctgctccc 7200
   ggcatccgct tacagacaag ctgtgaccgt ctccgggagc tgcatgtgtc agaggttttc 7260
   accgtcatca ccgaaacgcg cgagacgaaa gggcctcgtg atacgcctat ttttataggt 7320
   taatgtcatg ataataatgg tttcttagac gtcaggtggc acttttcggg gaaatgtgcg 7380
   cggaacccct atttgtttat ttttctaaat acattcaaat atgtatccgc tcatgagaca 7440
   ataaccctga taaatgcttc aataatattg aaaaaggaag agtatgagta ttcaacattt 7500
   ccgtgtcgcc cttattccct tttttgcggc attttgcctt cctgtttttg ctcacccaga 7560
   aacgctggtg aaagtaaaag atgctgaaga tcagttgggt gcacgagtgg gttacatcga 7620
   actggatctc aacagcggta agatccttga gagttttcgc cccgaagaac gttttccaat 7680
   gatgagcact tttaaagttc tgctatgtgg cgcggtatta tcccgtattg acgccgggca 7740
   agagcaactc ggtcgccgca tacactattc tcagaatgac ttggttgagt actcaccagt 7800
   cacagaaaag catcttacgg atggcatgac agtaagagaa ttatgcagtg ctgccataac 7860
   catgagtgat aacactgcgg ccaacttact tctgacaacg atcggaggac cgaaggagct 7920
   aaccgctttt ttgcacaaca tgggggatca tgtaactcgc cttgatcgtt gggaaccgga 7980
   gctgaatgaa gccataccaa acgacgagcg tgacaccacg atgcctgtag caatggcaac 8040
   aacgttgcgc aaactattaa ctggcgaact acttactcta gcttcccggc aacaattaat 8100
   agactggatg gaggcggata aagttgcagg accacttctg cgctcggccc ttccggctgg 8160
   ctggtttatt gctgataaat ctggagccgg tgagcgtggg tctcgcggta tcattgcagc 8220
   actggggcca gatggtaagc cctcccgtat cgtagttatc tacacgacgg ggagtcaggc 8280
   aactatggat gaacgaaata gacagatcgc tgagataggt gcctcactga ttaagcattg 8340
   gtaactgtca gaccaagttt actcatatat actttagatt gatttaaaac ttcattttta 8400
   atttaaaagg atctaggtga agatcctttt tgataatctc atgaccaaaa tcccttaacg 8460
   tgagttttcg ttccactgag cgtcagaccc cgtagaaaag atcaaaggat cttcttgaga 8520
   tccttttttt ctgcgcgtaa tctgctgctt gcaaacaaaa aaaccaccgc taccagcggt 8580
   ggtttgtttg ccggatcaag agctaccaac tctttttccg aaggtaactg gcttcagcag 8640
   agcgcagata ccaaatactg ttcttctagt gtagccgtag ttaggccacc acttcaagaa 8700
   ctctgtagca ccgcctacat acctcgctct gctaatcctg ttaccagtgg ctgctgccag 8760
   tggcgataag tcgtgtctta ccgggttgga ctcaagacga tagttaccgg ataaggcgca 8820
   gcggtcgggc tgaacggggg gttcgtgcac acagcccagc ttggagcgaa cgacctacac 8880
   cgaactgaga tacctacagc gtgagctatg agaaagcgcc acgcttcccg aagggagaaa 8940
   ggcggacagg tatccggtaa gcggcagggt cggaacagga gagcgcacga gggagcttcc 9000
   agggggaaac gcctggtatc tttatagtcc tgtcggg 9037

<210> 2
<211> 66
<212> DNA
<213> Artificial sequence

<220>
<223> Description of artificial sequence:IRES sequence

<400> 2
ctgcaggtcg agccggcggg tttctgacat ccggcgggtg actcacaacc ccagaaacag 60
acatcc 66

## Description

The invention relates namely to a biotinylation process, to vectors for expression of biotinylated proteins in mammalian cells *in vivo,* and their use for identification of protein-protein and protein-nucleic acid interactions *in vivo.*

With completion of the Human Genome Project, the focus of research is moving towards the systematic study of the human proteome. In particular, the identification and cataloging of the protein-protein and protein-DNA/RNA interactions is expected to complement the one-dimensional information contained in the human genome sequence. It will contribute to reconstruction of the three dimensional supramolecular picture of a human cell and will also help in understanding the function of a particular protein, as well as in identification of novel drug targets. One particular aspect of the human proteome research concerns information about the interaction partners (protein and DNA/RNA) of a particular protein. However, compared to DNA, proteins have much more variable chemical and physical properties. Therefore, the high throughput data mining in proteomics requires generalized approaches independent as much as possible on the specifics of a particular protein. Several methods exist to identify novel protein interaction partners, and to study protein-protein interactions and protein-DNA/RNA interactions.

### Protein-protein interactions

1. Historically first method to study protein-protein interaction is to purify the native complexes of the proteins directly from the cells and identify their components. The methods of biochemical separation, such as chromatography, centrifugation, etc. could be used for this purpose, provided that the presence of the complex in the fractions could be detected in some assay. If the function of the complex is known, such an assay could be based on its enzymatic activity, or on the ability to bind a particular ligand. Alternatively, the purification scheme could include, as a crucial step, affinity chromatography with antibodies (some time obtained serendipitously, as in the case of autoantigen antibodies) against one of the compenents of the complex. In some cases, if the ligands of the protein or the complex are known, the affinity chromatography could be performed with the ligand, instead of the antibodies. The identification of the components of the complex can be accomplished by a number of methods developed in the field of proteomics, such as mass-spectrometry or Edman-degradation sequencing.
   The main limitation of this approach is that it is not easily adaptable to the high throughput format, needed for modem proteomics. The only version of this approach independent on the *a priori* knowledge of the function of the complex (or its components) is based on the use of antibodies against some components of the complex. However, the development of specific and sensitive antibodies is an expensive and long procedure. This limitation precludes development of this approach into a high throughput format. Also, the affinity purification of a protein complex with antibodies against a native protein has an additional problem. Often competition between the antibody and an interaction partner for binding to identical (or physically proximal) domains of the studied protein will make the affinity purification procedure incompatible with identification of this particular interaction partner. This limitation is the most significant when polyclonal antibodies are used, which usually recognize many different epitopes on the protein.
   Two other classes (types) of approaches, currently used for identification of interaction partners, allow for a systematic high throughput analysis of many proteins and therefore are suitable for the proteomics needs. However, they have their own limitations.
2. The two-hybrid system , 2, 3. This approach allows for *in vivo* detection of the protein-protein interactions by fusing two putative partner proteins to two functional domains of a particular signal transducing protein (STP), most commonly a transcription factor, whose activity is easily detectable in a particular experimental setup. For the signal transduction to occur, these two STP domains have to be in a close physical proximity, which will be provided in the case of the interaction between the protein partners. To search for novel protein partners, one constructs an expression library wherein each ORF is fused with an STP domain. This library is then transformed into the cells that express the protein of interest fused with the second STP domain. The clones that express the putative interaction partners of the protein of interest are selected based on the functional assay for the particular STP.
3. The affinity chromatography with immobilized protein of interest. In this setup, the protein of interest is immobilized on a solid chromatographic support, either by direct chemical crosslinking, or via a fusion with such widely used interacting protein domains as GST, His etc. The immobilised protein is incubated with a protein mixture (tissue, cellular, nuclear extract or others). After extensive washing, the retained polypeptides are identified by a number of methods developed in the field of proteomics, such as mass-spectrometry or Edman-degradation sequencing.

These two generic approaches have namely several common limitations. First, they are designed to detect mostly binary interactions. However, often the physiologically relevant interactions are not of a binary type (exemplified by receptor-ligand interactions) but rather are represented by large multiprotein complexes 4, 5. In these complexes, multiple interactions between different subunits could contribute to the stability of an overall complex. Also, additional subunits can affect the strength of a particular binary interaction by allosteric effects. Therefore, the methodology designed to hunt only for the binary partners will miss this kind of interactions quite widespread in the cell.

Second limitation of both approaches is that they are designed to pick only *potential* interaction partners from the pool of presented canditates. Whether these potential partners reflect the actual interactions that occur in the cell has to be confirmed by *in vivo* analysis of the homologous (e.g. mammalian) cell system. Usually it is achieved by coimmunoprecipitation method, which requires antibody development, with all problems the accompanying this work.

### Protein-DNA/RNA interactions

For the search of novel protein-DNA/RNA interactions, the approaches, adaptable for a high throughput analysis, can be broadly divided into two classes: *in vivo* and *in vitro* approaches.
1. Generic *in vivo* approach, based on transformation of cells with a library of sequences in a setup that allows to select cells according to a particular phenotype, that depends on the interaction between probed DNA and protein 6.
2. Generic *in vitro* approach (affinity chromatography) - immobilization of the protein (or nucleic acid) of interest on chromatography support and incubation with a nucleic acid (or protein) mixture.

The limitations here are very similar to the previous case: 1. Both approaches are designed to detect mostly binary interactions (in particular, they do not take into account chromatin context of the DNA). 2. They can find only potential binding sites.

As in the previous case, purification of protein-DNA/RNA complexes directly from the cells is the approach that lacks most of the limitations of the previous techniques, as it is designed to look for the actual interaction as they exist in the cell. One such approach is Chromatin ImmunoPrecipitation (ChIP) 7, 8. In one version, X-ChIP, the DNA is crosslinked *in vivo* to bound proteins, then sheared and the nucleoprotein complexes are immunoprecipitated with antibodies directed against the studied proteins. If the putative DNA target of the protein is known, the interaction can be detected by PCR. Lately, ChIP was used to identify unknown *in vivo* targets.

As one might expect from the previous discussion, the main disadvantage of ChIP is the need to develop highly specific and sensitive antibodies for each studied protein. Due to the generally high nonspecific binding of chromatin to surfaces and chromatographic support, the quality requirement for the antibodies is even higher than in the case of protein complex purification. Also, the problem of antibody -DNA competition for binding to the epitope-binding site is the same as in the previous discussion. These limitations preclude the adaptation of the ChIP assay in the original form for the high throughput search of DNA targets of a protein of interest.

The invention allows to solve the technical problems of the prior art and to obtain modified peptides that allow a reliable study *in vivo* of the interaction between protein and protein interaction partners (IP).

In this purpose, the inventors have developed an Epitope tagging technique. Epitope tagging is known in the prior art as a technique comprising protein modification (usually by recombinant DNA technology) that adds to the native protein a short peptide sequence (epitope tag), recognizable by a specific antibody 9. The epitope tag can be put in any place of the protein, usually on its N- or C-terminus. As a part of this approach, recombinant cell lines are constructed that express the epitope tagged protein. The protein of interest can be then detected and purified with the antibody directed against the epitope tag. One limitation of the known epitope-tag system is a possibility of the epitope-tag to affect the function and interaction characteristics of the protein of interest.

So far, epitope-tagging systems used in mammalian cells for detection, localization and purification of proteins (Flag-, HA-, myc-tags 11 , 13) are not enough efficient due to the unsufficient interaction of the epitope tag and antibody. As a result, usually high nonspecific background is observed during purification of complexes. This is due to the much higher complexity of mammalian cells compared to the cells of simpler organisms, very low levels of expression of many proteins and the need to use large amounts of starting material for affinity purification, which increases the amount of undesirable proteins sticking nonspecifically to the chromatographic solid phase.

In order to solve this problem, the inventors have used biotine as epitope. The biotin-avidin interaction is one of the strongest and most specific interactions used in molecular biology 14. Its dissociation constant is 10⁻¹⁵ M, which is several orders of magnitude stronger than for most of antibodies.

The inventors have used a protein biotinylation technique. The inventors have used the biotin acceptor domains for the identification of the protein-protein or protein-nucleic acid interactions *in vivo,* by copurifying the complexes of the biotinylated proteins of interest with the interaction partners (proteins or DNA/RNA) and identification of these partners.

Such engeneering of proteins to target them for biotinylation can be considered a version of epitope-tagging, as it shares most of its features, except that the tag is recognized not by an antibody but a (strept)avidin moiety. Very few proteins in the cell are biotinylated naturally, therefore, purification and detection of a given biotinylated protein should be very specific as well.

In the prior art, biotinylation was performed *in vitro* using chemical crosslinking. In the last decade, it was demonstrated that fusion of a biotin acceptor domain of naturally biotinylated proteins with the protein of interest leads to its *in vivo* biotinylation 15. Short (around 15aa) peptides that are good biotinylation substrates of the bacterial BirA enzyme can be also fused with the protein of interest and make it a biotinylation target 16. In E.coli and related cells, the proteins fused to the biotin acceptor domains are already biotinylated *in vivo,* due to the endogenous BirA enzyme activity. If this activity is limiting (in the case of overproduction of the protein in the host cells, or use of heterologous system) BirA can be coexpressed in the cells together with the protein of interest, which allows to achieve high levels of biotinylation *in vivo.* In addition, free biotin (vitamin H) can be directly added to the medium to help the biotinylation.

The advantage of the biotinylation tag, compared to other tags for the particular task of the complex purification, is based on the strength and specificity of the biotin-avidin interaction.

The inventors had to solve not only the problem of using a short tag that does not disrupt the function of the protein of interest, but also to obtain an appropriated coexpression of the biotinylation enzyme (for example, BirA) and of the protein of interest in the same cells. One potential problem with the BirA expression is the large volume of the mammalian cells compared to the bacterial cells, which would negatively affect the efficiency of biotinylation. The amounts of BirA required to achieve efficient *in vivo* biotinylation might negatively interfere with the cell functioning, for example, due to undesirable biotinylation of other proteins in the cell. This will also lead to higher nonspecific background during purification.

The inventors have achieved the 100% biotinylation efficiency, at the same time keeping the levels of the enzyme on the minimal necessary level.

The inventors have succeeded to get efficient biotinylation, using physiologically (low) biotin concentration in the mammalian cell growth medium, less than 1mg/L, and typically 0.2mg/L to 0.033mg/L.

It is accomplished by coexpressing the protein of interest and the biotinylating enzyme from the same mRNA.

This increases the chance of the enzyme-target interaction in large mammalian cell and thus minimizes the amounts of the enzyme needed to achieve 100% biotinylation of the protein of interest. Unlike in the procaryotes, usually only one protein can be synthesized from one mRNA in the eukaryotic cell. Coexpression of several proteins in mammalian cells from the same mRNA requires high level of expertise.

The inventors accomplished this task by using an IRES sequence (Internal Ribosome Entry Site).

Thus, the invention relates according to a first aspect to a method for expressing in eucaryote cells a modified target protein of interest comprising expressing (i) a protein of interest and (ii) a modifying enzyme that achieves the modification of said target protein, wherein the target protein of interest and the modifying enzyme are coexpressed from the same RNA. The vector used for the expression of the modifying enzyme from the same message RNA as the target protein comprises an Internal Ribosome Entry Site (IRES).

In a preferred embodiment the modifying enzyme is a biotinylating enzyme that achieves biotinylation of the protein of interest, and the target protein is fused to at least one biotin acceptor domain (BAD). The vector used for efficient biotinylation of the protein of interest *in vivo* in mammalian cells, preferably contains as essential part, BAD domain and BirA, driven by IRES.

Thus the invention provides a method to express *in vivo* in mammalian cells biotinylated versions of the proteins of interest. The inventors have used a set of vectors that express the protein of interest fused with a short biotin acceptor domain. These vectors also express biotinylation enzyme BirA, required for biotinylation of this domain *in vivo.*

The IRES are known from the one skilled in the art (1988, Pettetier and Sonenberg, Nature, 334:320-325) : particular virus mRNAs containing specific sequences internal to the mRNA are translated by an unusual mechanism of "internal ribosome binding" that enable a ribosome to bind and initiate translation. The sequences are termed "Internal Ribosome Entry Site" (IRES). The eukaryotic ribosomes recognise IRES. The IRES used in the present invention can be derived from either a viral or cellular gene that undergoes cap-independent translation. Preferably, the IRES is derived from a picomavirus, such as polio virus or encephalomyocarditis virus.

According to a realization, the biotinylated protein of interest is expressed via transient transfection.

According to an other realization the biotinylated protein of interest is expressed via generation of stably expressing cell lines. The biotinylated protein of interest is typically expressed from a vector allowing an inducible expression.

Preferably the transfected cells are from a tissue sample, and the tissue sample is mammalian, preferably human (eventually biopsied sample).

According to an other aspect the invention relates to a method for intracellular localization of the biotinylated protein.

In a realization the localization is determined using incubation of cells with streptavidin/avidin conjugated to a detectable marker (fluorescent, radionucleide etc...) and observed in a microscope.

In an other realization the localization is determined via incubation of the sample with streptavidin/avidin coated gold beads and observed in electron microscope.

According to an other aspect the invention relates to a method for in vivo labeling of biotinylated protein typically by incubating the cells expressing the biotinylated protein in the presence of radioactive biotin.

In a realization the labeled protein is detected in fractions after biochemical separation by autoradiography.

In an other realization the biotinylated protein is detected in fractions after biochemical separation via its interaction with avidin (streptavidin). The detection is based on western blot analysis.

According to an other aspect the invention, the biotinylated protein is purified from the cells.

In a realization the biotinylated protein is purified by affinity chromatography with the immobilised streptavidin, avidin, its derivatives (i.e. monomeric avidin) or antibiotin antibodies. For instance the avidin derivative is monomeric avidin.

The purification of the biotinylated protein may comprise an additional affinity chromatography step. The additional chromatography step is for instance 6xHis tag.

According to an other aspect the invention, the biotinylated protein is purified in a complex with interaction partners of the protein, typically other polypeptides and nucleic acids.

In an realization the said complex biotinylated protein is purified in mild conditions. In an other realization the cells are treated with crosslinking reagents and the said complex is purified in stringent conditions. The results are compared with a positive control, typically a known protein.

The complex can be further analysed with biochemical, mass-spectrometrical and other methods.

In a realization this further analysis of the interaction partners is performed directly on a chomatographic support, without further elution of the protein of interest or/and the interaction partners.

In an other realization the further analysis is performed after removal of the chromatographic support ; for instance the epitope-tag includes a protease cleavage site and the chromatographic support is removed by a protease cleavage.

The complex can also be assayed on enzymatic activity, such as histone acetyltransferase, kinase, methyltransferase. The effect of the interaction partners on the enzymatic activity of the protein of interest is assayed with a high precision compared to the prior art methods..

The complex can be also analysed on the presence of interaction partners.

In a realization this presence is monitored by western analysis with corresponding antibodies (Colmmunoprecipitation).

In a realization the pattern of protein interaction partners is visualized via one or two dimensional polyacrilamide gel separation.

In a realization, the gel is stained with silver or coomassie or fluorescent stains such as Sypro Ruby, to detect the protein interaction partners. Alternatively the protein interaction partners are chemically biotinylated before separation and visualised on a westerm blot with streptavidin, avidin and other biotin-binding moities conjugated with a detectable label (such as peroxidase, phosphatase, FITC etc).

In a preferred embodiment the protein interaction partners of the *in vivo* biotinylated protein are identified by mass- spectrometry. Said mass-spectrometrical identification can be performed after gel separation of the interaction partners, or after direct protease digestion of the complex, separation of the complex mixture of peptides with one or two-dimensional mass-spectrometry and identification of the peptides with mass-spectrometer.

Said digestion is performed directly on the chromatograhic support, without prior elution of material, or after elution of the interaction partners in conditions that disrupt the interaction of the said partners with the biotinylated protein.

In a realization said digestion is performed after elution of the interaction partners in conditions that disrupt the interaction of the biotinylated partner with chromatographic support. Preferably the said conditions are 8M Guanidine HC1 with pH 1.5

In a realization the epitope tag includes a protease cleavage site and the complex is released after protease digestion. Preferably the said protease is TEV (Tobacco Etch Virus).

According to an other aspect, the *in vivo* interaction of the biotinylated protein with DNA/RNA, is monitored by purification of the said protein complexed with DNA/RNA directly from the cells.

The protein is typically crosslinked *in vivo* to DNA/RNA via chemical or other crosslinking method. Typically the crosslininking reagent is formaldehyde. The presence of DNA/RNA in the immunoprecipitation is detected by PCR.

In a realization RNA is converted to cDNA by reverse transcription before PCR amplification.

In a realization the presence of DNA/RNA in the immunoprecipitation is detected by DNA array hybridization.

In a realization the said DNA/RNA in the immunoprecipitation is labeled by LM-PCR.

The said DNA/RNA in the immunoprecipitation is directly labeled without prior amplification with PCR.

In a realization the DNA/cDNA obtained after biotin affinity purification is cloned. DNA is cloned directly or after PCR amplification.

According to an other aspect the invention relates to a nucleic construct comprising a DNA sequence encoding a protein of interest sequence, an epitope tag, a DNA sequence coding for a modifying enzyme, an IRES sequence.

Preferably the nucleic construct comprises a DNA sequence encoding a protein of interest, a BAD domain, a BirA sequence, an IRES sequence capable of conducting the coexpression from the same RNA of the protein of interest fused with the BAD domain, and of the BirA.

The invention also relates to a vector for efficient modification, namely for efficient biotinylation, of a protein of interest *in vivo* in mammalian cells, comprising such a nucleic construct.

The invention also relates to an eucaryote host cell comprising said vector, preferably mammalian host cells, most preferably, human host cells. Suitable host cells include human fibroblast cell lines (e.g., HeLa cells, 293T cells, 293 cells, W238 cells and HOS cells), human T-cells (e.g., Jurkat, CEM and H9 cells), monkey cell lines (e.g., CV1 cells and COS cells), rodent cell lines (e.g., 3T3 cells, L cells, C127 cells and

BHK cells). The constructs can be introduced using any of a variety of techniques and agents, including electroporation, calcium phosphate precipitation, DEAE-dextran, lipofectin, cationic lipids and viral (e.g., retroviral) infection.

The invention also relates to a kit (A) for transient expression of protein of interest *in vivo,* comprising:
1) the vector for transient transfection,
2) the transfection reagent,
3) means for sorting the transfected cells, typically magnetic beads coupled with antiIL2R antibodies.

The invention also relates to a kit (B) for generation of stable cell lines expressing the protein of interest *in vivo,* comprising:
- the retroviral vector for transient transfection,
- packaging cell line,
- the transfection reagent,
- means for sorting the transduced cells, typically magnetic beads coupled with antiIL2R antibodies.
- ptionally human cell lines (HeLa, 293 and other) that express a mouse protein that allows the use of ecotropic packaging cell lines for transduction.

The invention also relates to a kit for *in vivo* localization of the protein of interest, comprising :
- A kit (A) or (B),
- Streptavidin or avidin or other biotin-interacting moieties conjugated with a fluorescent or color-generating label, such as FITC, Rhodamin, Peroxidase .

The invention also relates to a kit for visualization of the protein interaction partners of the protein of interest, comprising
- A kit (A) or (B),
- Extraction buffer,
- " Immobilized streptavidin or avidin or other biotin-interacting moieties, for purification of the protein in complex with the interaction partners,
- Activated biotin.
- Streptavidin or avidin or other biotin-interacting moieties conjugated with a detectable label, for detection of the biotin-labeled interaction partners,
- Optionally - TEV protease, if the vector contains the TEV-protease cleavage site. The invention also relates to a kit for identification of the protein interaction partners of the protein of interest, comprising
- a kit (A) or (B),
- Extraction buffer,
- Immobilized streptavidin or avidin or other biotin-interacting moieties
- Trypsin for protease digestion.

The interaction partners are identified by mass-spectrometry.

The invention also relates to a kit for detection of the DNA-protein interaction *in vivo* comprising:
■ a kit (A) or (B),
■ Crosslinking reagent,
■ chromatin preparation buffer,
■ Immobilised streptavidin or avidin or other biotin-interaction moieties,
■ PCR-amplification reagents,

The invention also relates to a kit for identification of DNA (RNA) targets *in vivo* comprising :
■ a kit (A) or (B),
■ Crosslinking reagent,
■ chromatin preparation buffer,
■ Immobilised streptavidin or avidin or other biotin-interaction moieties,
■ PCR-amplification reagents,
■ Cloning kit or kit for fluorescent labeling for DNA array identification.

The invention also relates to a method for screening a test compound for its ability to modulate interaction between a protein and a protein interaction partner comprising
i) preparing a nucleic construct described above,
ii) introducing said construct into a vertebrate host cell and culturing said host cell in the presence and absence of said test compound, and
iii) measuring the level of interaction of the interaction partner with the biotinylated protein of interest in the presence and absence of said test compound, a difference in the level of expression of said indicator gene in the presence of said test compound, as compared to the level of biotinylation in the absence of said test compound, being indicative of a test compound that modulates interaction of said protein with said protein interaction partner.

The term "antibody" refers to antibodies and antibody fragments that retain the ability to bind the epitope that the intact antibody binds, whether the antibody or fragment is produced by hybridoma cell lines, by immunization to elicit a polyclonal antibody response, or by recombinant host cells that have been transformed with a recombinant DNA expression vector that encodes the antibody or antibody fragment. The term "antigen" is defined as a molecule that induces the formation of an antibody or is capable of binding specifically to the antigen-binding sites of an antibody. The term "biotinylation enzyme" refers to the class of enzymes known as biotin protein ligases, or enzymes which biotinylate other proteins or peptides. The term "epitope" refers to that portion of an antigen that interacts with an antibody. The term "host cell" refers to a eukaryotic cell or group of cells that can be or has been transformed by a recombinant DNA vector.

The term "operably linked" refers to the placement of one nucleic acid into a functional relationship with another nucleic acid. For instance, a promoter is "operably linked" to a coding sequence if the promoter causes the transcription of the coding sequence. Generally, "operably linked" means that the DNA sequences being linked are contiguous and, where necessary to join two peptide or protein coding regions, in reading frame with one another. The term "peptide" refers to an oligomer in which the monomers are amino acids (usually alpha-amino acids) joined together through amide bonds. Alternatively, a "peptide" can be referred to as a "polypeptide." Peptides are more than two amino acid monomers long, but more often are more than 5 to 10 amino acid monomers long and can be even longer than 20 amino acids, although peptides longer than 20 amino acids are more likely to be called"polypeptides."

The term "protein" is well known in the art and usually refers to a very large polypeptide, or set of associated polypeptides, that has some biological function. For purposes of the present invention the terms "peptide," "polypeptide," and "protein" are largely interchangeable as libraries of all three types can be prepared using substantially similar methodology.

The terms "recombinant DNA cloning vector" and "recombinant DNA expression vector" refer to a DNA or RNA molecule that encodes a useful function and can either be used to transform a host cell or be introduced into a cell-free translation system to produce a protein encoded by the vector. For purposes of the present invention, a cloning vector typically serves primarily as an intermediate in the construction of an expression vector; the latter vector is used to transform or transfect a host cell (or is introduced into a cell-free transcription and translation system) so that the transformed host cell (or cell-free transcription and translation system) produces a protein or other product encoded by the vector. Such vectors are typically "plasmids," which, for purposes of the present invention, are vectors that can be extrachromosomally maintained in a host cell, but can also be vectors that integrate into the genome of a host cell.

Other aspects and advantages of the invention will result from the detailed description illustrated by the drawings :

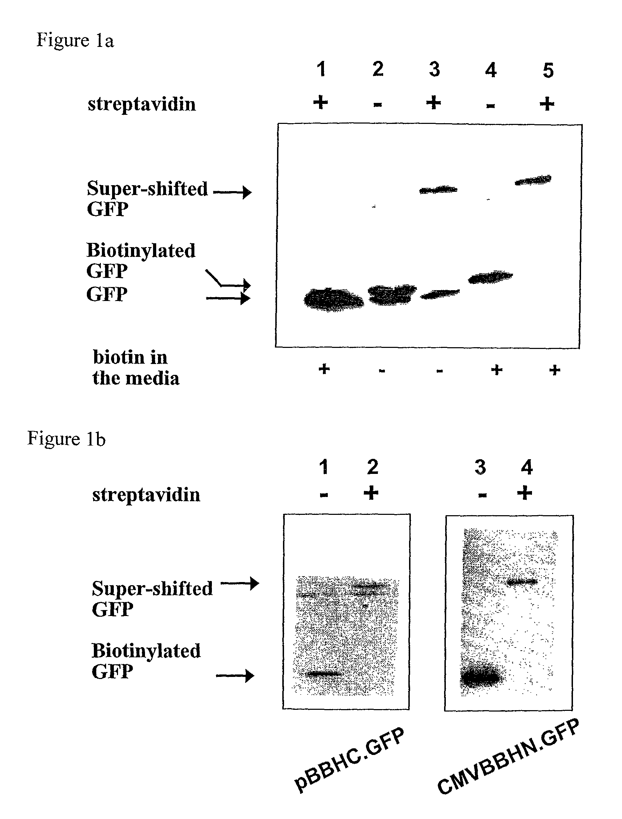

- Figure 1 illustrates the 100% biotinylation of GFP *in vivo* with different BAD vectors.
   a. In stable expression format. Hela cells, transduced with pBBHN.GFP vector, were incubated in DMEM medium (that contains no biotin, lanes 2, 3) or RPMI (with 0.2mg/L biotin, lanes 4,5). The extracts were analysed on western with anti-GFP antibodies. To detect biotinylated GFP, 200ng of streptavidin was added to the sample in the lanes 1,3 and 5. Lane 1 corresponds to the cells transduced with pBN.GFP vector that does not have BirA gene.
   b. Transient expression format: HEK 293 cells were transfected with pBBHC.GFP (lanes 1,2) or CMV.BBHN.GFP (lanes 3,4) and analysed as in 1a.

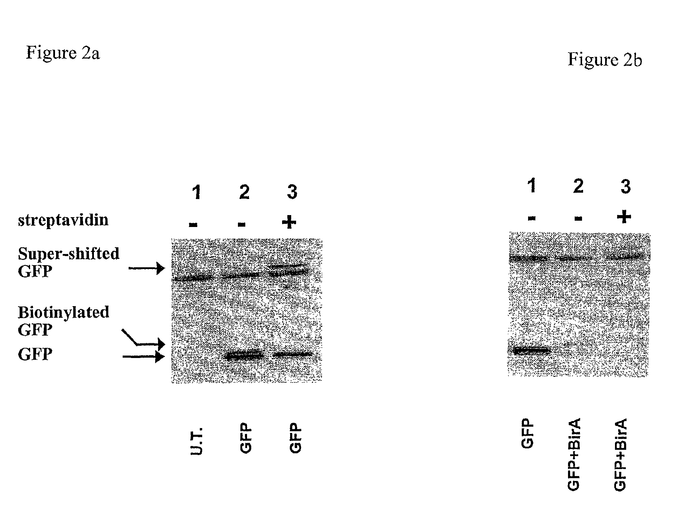

- Figure 2 shows that trans-biotinylation is not efficient:
   a. stably integrated BirA does not biotinylate with 100% efficiency.
      HEK293 cells, transduced with pBBHN.TAP54 vector, were transiently transfected with a pBN.GFP plasmid (expressing GFP BAD-domain fusion without BirA). They were further analysed as in 1a.
   b. co-transfection of the target gene and BirA lead to decrease of the protein expression.
      HEK 293 cells were transiently cotransfected with pBN.GFP and CMV.BirA. They were further analysed as in 1a.

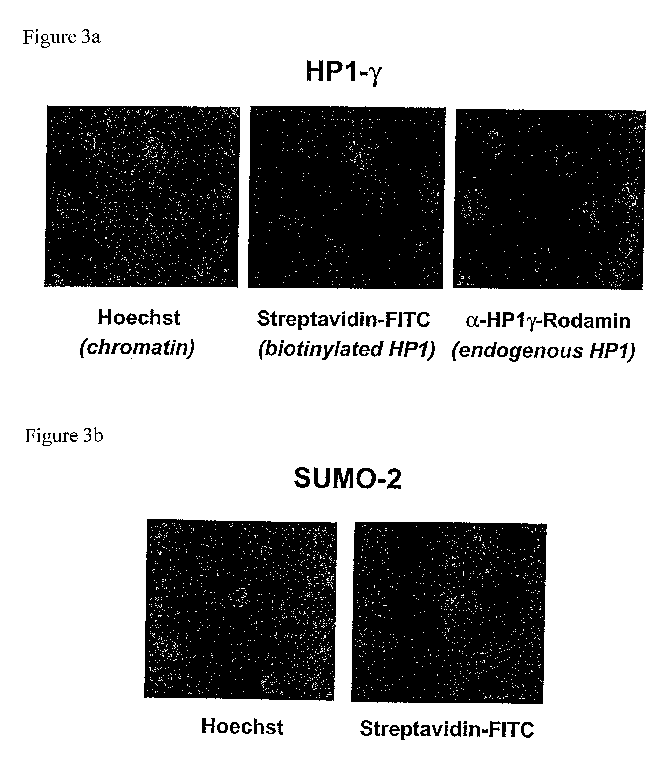

- Figure 3 shows that HP 1 localization is not disturbed by *in vivo* biotinylation.
   3.a. HP1 localisation : 3T3 cells were transfected with pBBHN.HP1g and double-stained with anti-HP1g antibodies and streptavidin, detected by Rhodamin or FITC, correspondingly. Chromatin was stained with Hoechst 33258. The pattern of staining of the cells with HP1 is exactly the same between chromatin, biotinylated HP1 and endogenous HP1.
   3.b. SUMO-2 localisation : 3T3 cells were transfected with pBBHN.SUMO2, stained with streptavidin-FITC and counterstained with Hoecht 33258. The pattern of staining is the same like for 3.a.

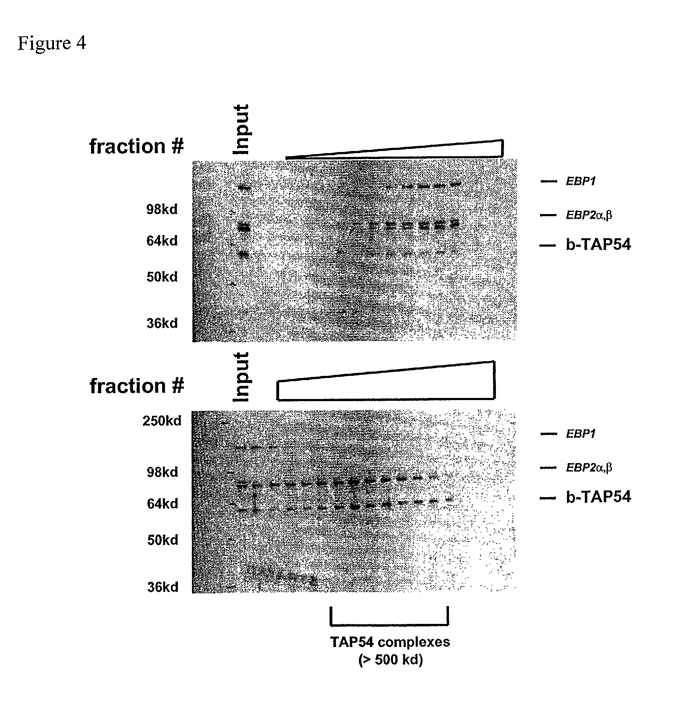

- Figure 4 shows that biotinylated TAP54 is present in large macromolecular complexes *in vivo.*
   Gel-filtration fractions from the biotinylated TAP54 expressing cells were analysed on western with streptavidin-FITC. The biotinylated TAP54 and endogenous biotinylated proteins are indicated by asteriscs. The molecular weghts acorresponding to the fractions are shown below. EBP means Endogenous Biotinylated Proteins (acetylCoA carboxylase, pyruvate carboxylase).

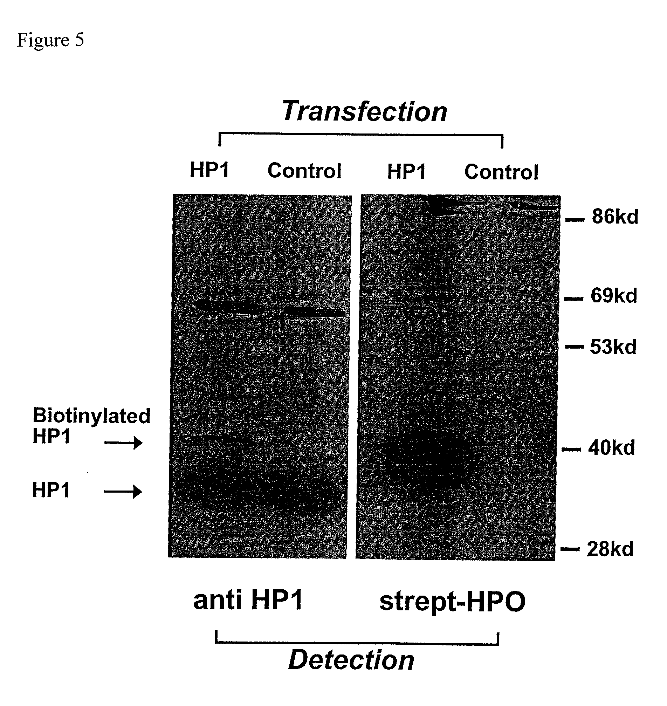

- Figure 5 shows that expression levels of the biotinylated proteins are lower than that of endogenous proteins.
   3T3 cells were transfected with pBBHN.HPla and the extract was analysed on western with antiHPl antibodies and with streptavidin-HPO. The endogenous HP1 and the biotinylated HP1 are indicated.

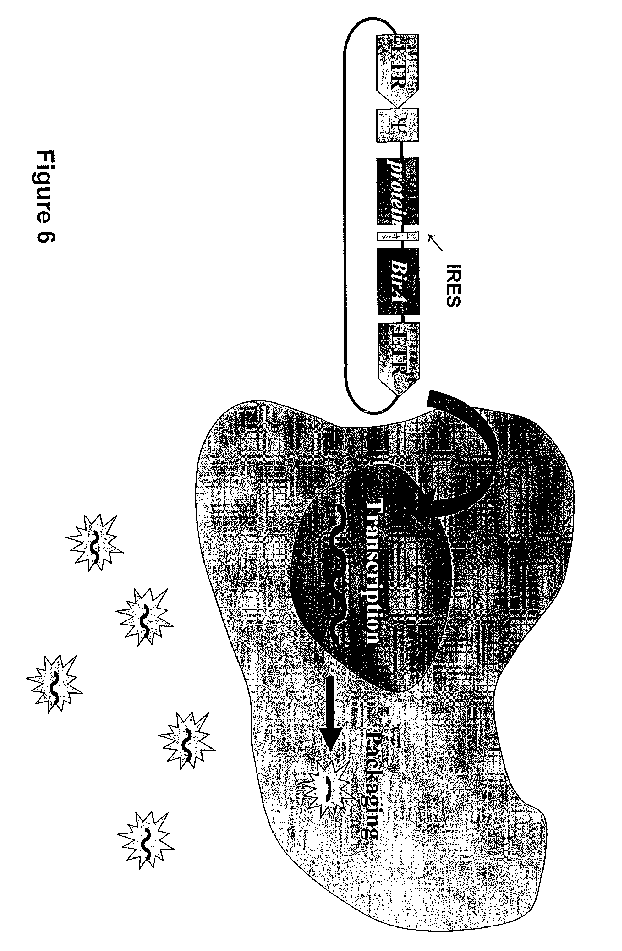

- Figure 6 illustrates transient transfection of cells : the vector containing the pattern LTR-ψ-protein of interest-IRES-BirA-LTR leads to the production of viral particles containing the DNA of interest.

The inventors constructed retroviral vectors that allow to insert an open reading frame of a protein of interest to express a fusion version of this protein, that bear, as a N- or C-terminal tag, a sequence that is efficiently biotinylated by a BirA enzyme. They designed two different versions of the vector that can be used for either N-terminal or C-terminal tagging. In another version of the same vectors they added a His tag, to use for double-epitope tag purification.

The ORF for the BirA enzyme is placed on the same vector downstream of the gene of interest and its expression from the same mRNA is ensured by an internal ribosome Entry sequence (IRES). This design allows to provide a high local concentration of the BirA enzyme in the proximity of the protein of interest, which would ensure efficient biotinylation with minimal amounts of the BirA enzyme.

An additional feature of this particular set of vectors is the third ORF encoding a selection marker IL2R, which is under control of a second IRES. This tricistronic design allows to minimize the number of steps and reagents necessary to generate the required cell line.

To prove that this design allows to achieve 100% biotinylation of a desired protein, they cloned a eGFP gene (Clontech) in one of the vectors, namely pBBHN described below, produced retroviral particles and used HeLa cells to generate the stably transduced cell lines. The cells were incubated in medium that does not contain biotin (DMEM), as well as in the medium that does contain 0.2 mg/L (RPMI). Then the cell extract was produced from 10 ml of cells and the presence of the GFP in the cells was detected by western. The degree of biotinylation was tested by the streptavidin gelshift. As evident from the Figure 1, 50% of the GFP is in the form that can strongly interact with streptavidin : this is in the medium without exogenous biotin (streptavidin +, biotin -). However, when the cells are incubated in the biotin containing medium, 100% of the GFP is in the form that strongly interacts with streptavidin. In summary, they achieved 100% biotinylation in this system.

The vector pBBHN used, referenced SEQ ID N°1 in the sequence listing (Retroviral vector for creation of stable cell lines expressing biotinylated protein of interest), comprises 9037 nucleotides as follows :
1) 606-2872: mouse genomic DNA, LTR, splice donor, packaging, pol-gag, splice acceptor sequences of the pMFG retroviral vector (constructed by Paul Robbins and Braydon Guild in Richard Mulligan's lab [see PNAS 89:11332 for citation])
2) 2881- 2955: BAD domain and 6XHis tag (the BAD peptide was patented by the Avidity company (www.avidity.com). This epitope tag is efficiently biotinylated by the BirA enzyme. (Schatz PJ. Biotechnology (N Y). 1993 Oct;11(10):1138-43).
3) 2956-2975: Xho, Nsi, NotI restriction sites containing polylinker.
4) 2976-3041: short IRES (The IRES 1 is a sequence described in the paper by Chappell et al Edelman GM, Mauro VP. (Proc Natl Acad Sci U S A. 2000 Feb 15;97(4):1536-41referenced SEQ ID N°2 in the sequence listing). It is a relatively short sequence that is, presumably, recognised by a 18S ribosomal RNA.
5) 3042-4010: BirA ORF (Howard et al, Gene. 1985;35(3):321-31).
6) 4136-4629: EMC IRES (IRES from encephalomyocarditis virus, widely used in the field for expression of polycistronic mRNA in mammalian cells. For example, see Morgan RA et al Nucleic Acids Res. 1992 Mar 25;20(6):1293-9.)
7) 4636-5454: IL2R ORF (alpha subunit of the the InterLeukin 2 Receptor. Its use for the magnetic affinity sorting first described in Padmanabhan R et al Anal Biochem. 1988 May 1;170(2):341-8.
8) 5456-6882: retroviral LTR and mouse genomic DNA from the pMFG vector, the rest is the Puc19 vector backbone

Other vectors can also be used and lead to good results :
a) : PBBHC : the same as pBBHN, except for the c-terminal placing of the BAD-tag.
b),c) : pBBN, pBBC : the same as pBBHN or pBBHC, except the 6Xhis tag is absent.
d),e): (CMV.BBHN), (CMV.BBHC) : they have the sequence 2881-5455 from pBBHN (pBBHN) containing BAD domain, BirA and IL2R. Instead of relatively weak retroviral MuMoLV enhancer, they have CMV enhancer, which significantly increases expression (more than 10 fold).

Other IRES sequences than the IRES sequence mentioned above may be used, and are in the field in the invention, now that the inventor have defined the method of biotinylation measurement.

The material and methods were as follows.

### Stable cell line generation and transient transfection:

All cells were grown in the Dulbecco's modified Eagle's medium (DMEM, Gibco) with 10% fetal bovine serum (FCS, PAN Biotech). For transient transfection, a standard calcium phosphate precipitation method was used, and the cells were analysed one or two days after transfection. For retroviral transduction, first packaging cell line Phenix-E was transfected with the plasmid DNA of the retroviral vector, in two days the retroviral supernatant was harvested, filter sterilised and added to the HeLa or HEK 293 cells. Two days after, the transduced cells were sorted using magnetic affinity beads coupled with anti-IL2R antibodies (Ogryzko et al, Cell 1998).

### Western analysis:

Total cell extract for the analysis was prepared using RIPA buffer (50mM Tris HC1 pH 8.0, 150mM NaCl, 1% NP40, 0.5% Na deoxyholate, 0.1% SDS, 1mM EDTA). First, cells were washed with phosphate buffered saline (PBS) and then incubated with RIPA buffer directly on the 6 well plates for 5 min. The extract was harvested and spun with the high speed on a microcentrifuge. The SDP-PAGE separation, transfer to a nitrocellulose membrane, blocking, incubation with antibody and ECL detection are according to a standard protocol, except that for the detection of biotinylated proteins the washes were performed in a more stringent conditions by adding 500mM NaCl to the washing buffer (PBS+0.1% Tween). The anti-GFP antibodies are from Clontech. The HP1 antibodies are from Euromedex. The streptavidin-HPO conjugate is from Sigma.

### Immunofluorescence:

NIH3T3 cells were grown and transfected on coverslips. Fixation was with 4% formaldehyde in PBS for 15 min at 4 C. Fixed cells were permeabilised with 0.5% Triton for 15', blocked with 1%BSA and 10%FBS and incubated with anti HP1g antibodies. Then they were washed with PBS and incubated with secondary antibody conjugated to rhodamine. For detection of biotinylated proteins the cells were incubated with streptavidin-FITC for 1 hr at 37C and washed with PBS. The cell were observed on the fluorescent microscope and the images were taken using a CCD camera.

### Gel-filtration:

The cells stably expressing the biotinylated TAP54 protein were extracted with buffer B (10% glycerol, 20mM Tris pH 8.0, 0.2mM EDTA, 0.1% Tween, 10mM bME, 400mM KCl, 0.25mM PMSF). 20 µl of the extract was applied to the SMART-size Superdex 200 and separated on the AKTA Purifier system. The column was precalibrated with ferritin, albumin and aldolase. The fractions were subjected to Western analysis.

The inventors get the main following results illustrated by the drawings:
1)100% biotinylation of GFP *in viv*o with different vectors:
   - Retroviral vector (planned to use for generation of stable cell lines) both N-terminal and C-terminal BAD domain fusions were tested and confirmed to give 100% biotinylation.
   - Vector for transient transfection with stronger CMV promoter, the n-terminal bad domain fusion, was tested and confirmed to give 100% biotinylation.
2) Transbiotinylation is not efficient
   - Transfection of BAD.GFP fusion in a cell line that expresses BirA gives only 10-20% biotinylation (figure 2a).
   - Cotransfection of BAD.GFP with CMV.BirA vector gives 100% biotinylation, however, the levels of GFP dramatically decrease, most probably due to the promoter competition (figure 2.b);

The inventors have also shown (figure 3) that biotinylation does not generally disturb ability of a protein to interact with other proteins, and that biotinylated HP 1 proteins colocalize with heterochromatin and with endogenous HP1 proteins, while other proteins, that are not expected to be in heterochromatin, show a different localization pattern.

Further, size fractionation of biotinylated TAP54 extracted from cells shows that it is present in large complexes.

Further, expression levels of the biotinylated proteins of interest are usually lower than the levels of the endogenous proteins.

And the inventors have made Western analysis of HP1 and MyoD expression.

The inventors have also shown, in comparison with the methods of the prior art, that purification of a protein complexes using biotinylation is more efficient, and that detection of nucleic acids (DNA) associated with biotinylated proteins is more efficient and more specific.

The invention provides the main following advantages compared to the state of the art.

Compared to the biotinylation approaches already known, the invention addresses an additional problem particular to the large eukaryotic cells - how to minimize potential negative impact of expression of the heterologous enzyme BirA in the mammalian cells, at the same time ensuring the 100% biotinylation of the desired protein. The inventors solve it by coexpressing the biotinylating enzyme from the same mRNA, thus increasing its local concentration exactly where it is needed (this allows to minimize the expression of the BirA enzyme, required to achieve 100% biotinylation of the protein). Being a one vector approach, this polycistronic design has an additional advantage, as it also minimises the time and effort necessary for generation of the recombinant mammalian cells.

Compared to the other approaches to the protein-protein or protein/DNA interaction identification, the advantages of the epitope tagging technique are practically the same as those of the native complex purification, as this system is the closest to probing the actual macromolecular interactions in the cell. In addition, it is easier to adapt to the hight throughput format, as it overcomes the limitations of the native complex purification approach, rooted in the antibody development and use. The protocol is significally less expensive, fast and easily standardized, as it requires only one antibody and the cDNA, instead of a protein. For many used epitope tags (Flag-, HA-, myc-tags etc) the corresponding antibodies are commercially available.

The additional features of epitope-tagging that make it particularly useful for the identification of interaction partners are the following:
1. Nonspecific binding of undesired material (proteins/DNA) to the chromatographic medium during complex purification can be controlled by comparing the results of the purification with so called mock purification, i.e. incubation of the same medium (antibodies, beads etc) with an extract from the parent cells that do not express the epitope-tagged protein. Similar control is difficult in the case of antibodies against native proteins, as it would require finding cells that do not express the protein.
2. As the epitope tag on the protein is not engaged in any of its physiologically relevant interactions, there is less competition between the antibody and the interaction partners for binding to the protein.

Further, the biotinylation technique used by the inventors allows :
a. Faster biochemical purification
   Compared to Flag or HA tag purification, that usually takes at least 5 hours of incubation, the binding of Biotin to avidin takes 5 minutes. This saves time, minimizes degradation losses, and what is particularly important for the complex purification, minimizes losses of interaction partners due to dissociation of complexes during long incubation times.
b. More economical
   It requires less starting material and less of the purification reagents, due to the much stronger affinity.
c. Wide range of reagents are available commercially for detection and purification of the biotinylated molecules.
d. Higher specificity
   - 1. Because less material is necessary to obtain the same amount of the complex, there is less undesirable protein present during the binding stage, therefore, less chance to get unspecific binding.
   - 2. In the case of crosslinking (X-ChIP), the purification procedure can afford much more stringent washes than usually (higher salt, detergent) due to the strength of the biotin /avidin interaction.

Further, unlike the prior art, the inventors use one vector to generate in one step mammalian cell lines expressing biotinylated proteins. The one vector approach saves time and effort. Further they use retroviral vector to generate cell lines expressing biotinylated proteins. The retroviral vectors also minimizes time and the efficiency of gene transfer is much higher compared to the most other vectors

Besides, the IRES used minimize the amounts of the BirA enzyme necessary for 100% biotinylation of the target protein. This deals with the problem of negative effect of the BirA overexpression on the cell physiology and of biotinylation of undesirable proteins that increases the background during complex purification. And the particular IRES used in the vector is smaller than other IRESes, commonly used (40 bp instead of 500). It helps to maximize the capacity of the retroviral construct and in general, minimize the vector size.

Most of potential applications relate to fast and efficient creation of mammalian cell lines that express a biotinylated version of protein of interest.

These cell lines then can be used for detection, localisation, and purification of the proteins of interest directly from mammalian cells. Also, these cell lines can be used for identification of interaction partners of the proteins of interest by affinity chromatography on streptavidin column. It can be done directly either from cellular (nuclear) extracts or lysates, from native or from crosslinked *(in vivo* or *in vitro)* material.

The invention shows significantly improved results in namely the detection of proteins, their localization *in vivo* (by fluorescent or electron microscopy), their labeling, the identification of interaction partners (proteins and nucleic acids) *in vivo* (by coimmunoprecipitation on immobilised streptavidin or avidin), the visualisation of set of interaction partners.

The invention allows an expression of efficiently biotinylated proteins, an expression of efficiently postranslationally modified proteins that require the modification for their function (phosphorylated, acetylated, methylated, ubiquitinated, sumoilated, protease cleaved, etc...).

### REFERENCES

1. Legrain P, Selig L. *FEBS Lett* 480, 32-6 (2000).
2. Tucker CL, et al *P.Trends Cell Biol* 11, 102-6 (2001).
3. Serebriiskii IG, et al *.Biotechniques* 30, 634-6 (2001).
4. Peterson CL, Logie *C.J Cell Biochem* 78, 179-85 (2000).
5. Alberts B. *Cell* 92, 291-4 (1998).
6. Sieweke M. *Methods Mol Biol* 130, 59-77 (2000).
7. Kuo MH, Allis CD *Methods* 19, 425-33. (1999).
8. Orlando *V.Trends Biochem Sci.* 25, 99-104. (2000).
9. Fritze CE, Anderson TR. *Methods Enzymol* 327, 3-16 (2000).
10. Ogryzko et al *Cell* 94, 35-44 (1998).
11. Ikura et al. *Cell* 102, 463-73 (2000).
12. Ren B, et all *Science* 290, 2306-9 (2000).
13. Rowan AJ, Bodmer WF. *Hum Mutat 9,* 172-6 (1997).
14. Wilchek M, Bayer EA. *Trends Biochem Sci* 14, 408-12 (1989).
15. Cronan JE Jr. *JBiol Chem* 265, 10327-33 (1990).
16. Cull MG, Schatz PJ. *Methods Enzymol.* 326:, 430-40 (2000).
17. Parrott MB, Barry MA. *Mol Ther* 1, 96-104 (2000).
18. Parrott MB, BarryMA. *Biochem Biophys Res Commun* 281, 993-1000 (2001).
19. Zhou P, et al *Mol Cell.* 3, 751-6 (2000).

## Claims

1. A method for expressing in eucaryote cells a modified target protein of interest comprising expressing (i) a protein of interest and (ii) a modifying enzyme that achieves the modification of said target protein, wherein the target protein of interest and the modifying enzyme are coexpressed from the same RNA.

2. A method according to claim 1 wherein the modifying enzyme is a biotinylating enzyme that achieves biotinylation of the protein of interest, and the target protein is fused to a biotin acceptor domain.

3. A method according to claim 1 or 2 wherein the vector used for the expression of the modifying enzyme from the same message RNA as the target protein contains an Internal Ribosome Entry Site (IRES).

4. A method according to claim 3 wherein the vector used for efficient biotinylation of the protein of interest *in vivo* in mammalian cells, contains as essential part, BAD domain and BirA, driven by IRES.

5. A method according to anyone of claim 2 to 4, wherein the biotinylated protein of interest is expressed via transient transfection.

6. A method according to anyone of claim 2 to 4 wherein the biotinylated protein of interest is expressed via generation of stably expressing cell lines.

7. A method according to claim 6 wherein the biotinylated protein of interest is expressed in a vector allowing an inducible expression.

8. A method according to claim 6 wherein the transfected cells are from a tissue sample.

9. A method according to claim 6 wherein the tissue sample is mammalian

10. A method according to claim 3 wherein the tissue sample is human.

11. A method according to anyone of claim 1 to 10 wherein the intracellular localization of the biotinylated protein is determined using incubation of cells with streptavidin/avidin conjugated to a detectable marker (flourescent, radionuclide).

12. A method according to anyone of claim 1 to 10 wherein intracellular localization of the biotinylated protein is determined via incubation of the sample with streptavidin/avidin coated gold beads.

13. A method according to anyone of claim 1 to 12 wherein the protein is *in vivo* labeled, typically by incubating the cells expressing the biotinylated protein in the presence of radioactive biotin.

14. A method according to claim 13 wherein the labeled protein is detected in fractions after biochemical separation by autoradiography.

15. A method according to claim 14 wherein the biotinylated protein is detected in fractions after biochemical separation via its interaction with avidin (streptavidin).

16. A method according to claim 14 wherein the detection is based on western blot analysis.

17. A method according to claim 3 wherein the biotinylated protein is purified from the cells.

18. A method according to claim 17 wherein the biotinylated protein is purified by affinity chromatography with the immobilised streptavidin, avidin, its derivatives (i.e. monomeric avidin) or antibiotin antibodies

19. A method according to claim 18 wherein the avidin derivative is monomeric avidin.

20. A method according to claim 17 wherein the biotinylated protein is purified in a complex with interaction partners of the protein, typically other polypeptides and nucleic acids.

21. A method according to claim 20 wherein the complex is further analysed with biochemical, mass-spectrometrical and other methods.

22. A method according to claim 17 wherein the complex is assayed on enzymatic activity, such as histone acetyltransferase, kinase, methyltransferase.

23. A method according to claim 17 wherein the complex is analysed on the presence of interaction partners.

24. A method according to claim 23 wherein the presence is monitored by western analysis with corresponding antibodies (CoIP).

25. A method according to claim 23 wherein the pattern of protein interaction partners is visualised via one or two dimensional polyacrilamide gel separation.

26. A method according to claim 21 wherein the protein interaction partners of the *in vivo* biotinylated protein are identified by mass- spectrometry.

27. A method according to claim 26 wherein the said mass-spectrometrical identification is performed after gel separation of the interaction partners.

28. A method according to claim 26 wherein the said mass-spectrometrical identification is performed after direct protease digestion of the complex, separation of the complex mixture of peptides with one or two-dimensional mass-spectrometry and identification of the peptides with mass-spectrometer.

29. A method according to claim 28 wherein the said digestion is performed directly on the chromatograhic support, without prior elution of material.

30. A method according to claim 28 wherein the said digestion is performed after elution of the interaction partners in conditions that disrupt the interaction of the said partners with the biotinylated protein.

31. A method according to claim 28 wherein the said digestion is performed after elution of the interaction partners in conditions that disrupt the interaction of the biotinylated partner with chromatographic support.

32. A method according to claim 28 wherein the epitope tag includes a protease cleavage site and the complex is released after protease digestion.

33. A method according to claim 20 wherein the *in vivo* interaction of the biotinylated protein with DNA/RNA, is monitored by purification of the said protein complexed with DNA/RNA directly from the cells.

34. A method according to claim 33 wherein the protein is crosslinked *in vivo* to DNA/RNA via chemical or other crosslinking method.

35. A method according to claim 33 wherein the presence of DNA/RNA in the IP is detected by PCR.

36. A method according to claim 35 wherein RNA is converted to cDNA by reverse transcription before PCR amplification.

37. A method according to claim 33 wherein the presence of DNA/RNA in the IP is detected by DNA array hybridization.

38. A method according to claim 37 the said DNA/RNA in the IP is labeled by LM-PCR.

39. A method according to claim 37 the said DNA/RNA in the IP is directly labeled without prior amplification with PCR.

40. A method according to claim 33 wherein the DNA/cDNA obtained after biotin affinity purification is cloned.

41. A nucleic construct comprising a DNA sequence encoding a protein of interest sequence, an epitope tag, a DNA sequence coding for a modifying enzyme, an IRES sequence.

42. A nucleic construct comprising a DNA sequence encoding a protein of interest, a BAD domain, a BirA sequence, an IRES sequence capable of conducting the coexpression from the same RNA of the protein of interest fused with the BAD domain, and of the BirA.

43. A vector for efficient modification of a protein of interest *in vivo* in mammalian cells, comprising a construct according to claim 41.

44. A vector for efficient biotinylation of a protein of interest in vivo in mammalian cells, comprising a construct according to claim 42.

45. An eucaryote host cell comprising a vector according to claim 43 or 44.

46. A kit performing the method of claim 5 for transient expression of protein of interest in vivo, comprising
• the vector for transient transfection,
• the transfection reagent,
• means for sorting the transfected cells, typically magnetic beads coupled with antiIL2R antibodies.

47. A kit performing the method of claim 6 for generation of stable cell lines expressing the protein of interest *in vivo,* comprising
• the retroviral vector for transient transfection,
• packaging cell line,
• the transfection reagent,
• means for sorting the transduced cells, typically magnetic beads coupled with antiIL2R antibodies.
• Optionally human cell lines (HeLa, 293 and other) that express a mouse protein that allows the use of ecotropic packaging cell lines for transduction.

48. A kit performing the method of claim 11 to 16 for *in vivo* localization of the protein of interest, comprising :
• A kit according to claim 46 or 47,
• Streptavidin or avidin or other biotin-interacting moieties conjugated with a fluorescent or color-generating label, such as FITC, Rhodamin, Peroxidase .

49. A kit performing the method of claim 20 for visualization of the protein interaction partners of the protein of interest, comprising :
• A kit according to claim 46 or 47,
• Extraction buffer,
• Immobilized streptavidin or avidin or other biotin-interacting moieties, for purification of the protein in complex with the interaction partners,
• Activated biotin.
• Streptavidin or avidin or other biotin-interacting moieties conjugated with a detectable label, for detection of the biotin-labeled interaction partners,
• Optionally - TEV protease, if the vector contains the TEV-protease cleavage site.

50. A kit performing the method of claim 26 for identification of the protein interaction partners of the protein of interest, comprising
• a kit according to claim 46 or 47,
• Extraction buffer,
• Immobilized streptavidin or avidin or other biotin-interacting moieties
• " Trypsin for protease digestion.

51. A kit performing the method of claim 33 for detection of the DNA-protein interaction *in vivo* comprising :
• a kit according to claim 46 or 47,
• Crosslinking reagent,
• chromatin preparation buffer,
• Immobilised streptavidin or avidin or other biotin-interaction moieties,
• PCR-amplification reagents,

52. A kit performing the method of claim 33 for identification of DNA (RNA) targets *in vivo* comprising :
• a kit according to claim 46 or 47,
• Crosslinking reagent,
• chromatin preparation buffer,
• Immobilised streptavidin or avidin or other biotin-interaction moieties,
• PCR-amplification reagents,
• Cloning kit or kit for fluorescent labeling for DNA array identification.

53. A method for screening a test compound for its ability to modulate interaction between a protein and a protein interaction partner comprising
i) preparing a construct according to claim 41 or 42,
ii) introducing said construct into a vertebrate host cell and culturing said host cell in the presence and absence of said test compound, and
iii) measuring the level of interaction of the protein of interest with the biotinylated protein in the presence and absence of said test compound, a difference in the level of expression of said indicator gene in the presence of said test compound, as compared to the level of biotinylation in the absence of said test compound, being indicative of a test compound that modulates interaction of said protein with said protein interaction partner.
